(19) Europäisches Patentamt / European Patent Office / Office européen des brevets

(11) **EP 3 863 023 A1**

(12) **EUROPEAN PATENT APPLICATION**

(43) Date of publication:
**11.08.2021 Bulletin 2021/32**

(51) Int Cl.:
*G16H 50/30* *(2018.01)*

(21) Application number: **20020060.8**

(22) Date of filing: **10.02.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(72) Inventors:
• **Pomakidou, Sofia**
  **151 27 Melissia Attica (GR)**
• **Theodoridis, Thomas**
  **565 33 Thessaloniki (GR)**

(74) Representative: **Lioumbis, Alexandros**
  **Thrasyvoulou 17**
  **15234 Halandri (GR)**

(71) Applicant: **Medinevo Ike**
  **565 33 Thessaloniki (GR)**

(54) **A METHOD FOR INJURY PREDICTION**

(57) A method for injury prediction or prevention of living parts comprising the steps of simulating the geometry of a living part in a computer system, simulating the behavior of the living part in the computer system, calculating a maximum performance of the living part without the occurrence of an injury by the computer system, feeding to the system a load applied on the living part, calculating a current state of the living part by the computer system, comparing the current state of the living part with the calculated maximum performance of the living part. The living part is either a soft tissue comprising a fluid and a solid part or among tendons, joints and bones.

Figure 2

## Description

The present invention relates to a method for injury prevention of living parts, either for humans or animals.

## Background

Preventing and diagnosing injuries are well-documented problems and there are many tests that can be used to identify the cause of an injury and what recovery process is necessary.

An Electrodiagnostic Evaluation includes an electroanalysis assessment to diagnose pain, numbness or weakness that involves the peripheral nervous system and the muscles. Consisting of non-invasive or minimally invasive procedures, the evaluation provides small electrical connections to the affected areas of the body. The test results to information about the ability of the muscles to respond to the stimulation and the rate at which an electrical impulse moves through the nerves, helping to develop treatment options. The first part includes a neural conduction study, while the second part, the electromyography or "EMG", analyzes the health of the muscles and nerves that control the muscles both in rest and muscle contraction. This approach is currently commercially applied and described in various documents, like US2019313934, WO2019097166 and CN203736191. However, this method has a little accuracy because the electro sound in muscle surface includes large amounts of noise that leads to mutate results.

In the field of X-Ray Radiology, advancements in X-ray image processing allows on-site tests to athletes and quick interpretation, results and explanation via a computer monitor station located right in the exam room, as described in CN202235414. However, X-Ray Radiology gives information for two-dimension muscle sector and if the injury area is in between the monitoring surfaces there is no option for validated results. Additionally, there are only binary answers either there is muscle break or not and no prediction or information about local tissue wear.

Especially female athletes are at risk of developing osteoporosis or bone loss. Double energy X-ray absorption for Bone Density Scanning (DEXA Scanning), described in EP0761166, is the most commonly used test for measuring mean density and DEXA tests are more accurate than typical X-rays, as a person would need to lose 20-30 percent of bone density before this appearing in an X-ray. In this option there are similar issues such as binarity in injuring areas and hypothetically muscle volumes that are not efficient in providing a realistic muscle model.

CN103926169 describes the VO2 Max/Anaerobic (Lactate) Threshold Testing refers to the maximum amount of oxygen that someone can use during intense training. The anaerobic threshold control, also called lactic acid threshold control, measures the point during exercise, which the lactate accumulates in the blood faster than the body can remove it. Athletes can increase the threshold of lactic acid through appropriate training. However, it is well known that tissue response may have a variation and Chemical and Biological indicators may be affected from many unknown reasons non predictable or reproduceable.

Currently, injury prevention is implemented through the collection and analysis of statistical data. Existing techniques are based on translating, predicting and evaluating large amount of data using machine learning techniques, as described in bibliography, for example in "Sports Medicine Statistics, An Issue of Clinics in Sports Medicine" (Hart,J.M, Thompson, S.R, 2018, Sports Medicine Statistics, An Issue of Clinics in Sports Medicine, 1st edition, Elsevier).

The comparison between previous and future states leads to ambiguous estimations regarding physical condition. These methodologies are multi-parametric, which refers to the increment of uncertainty. Another limitation is the difference among people. The same training cycle may improve an athlete while harming another one. Statistical methods cannot make this distinction.

Based on the above, the need for performing monitoring with mechanical ways is evident. This calls for personalized medicine based on individual tissue geometry and mechanical properties for every single person. The field of Biomechanics offers an overall explanation for muscle function and suggests holistic treatments.

## Summary

The human muscular system is built to last a lifetime, thus this invention is related to failures that occur mainly to people or animals who intensively exercise under certain, hard conditions, or to people who suffer from injuries.

The present disclosure aims to provide a solution to the above problems and contribute to the prevention of injuries by implementing the disclosed method.

To this aim, it is disclosed a method for injury prediction of living parts comprising the steps of:

- simulating the geometry of a living part in a computer system,
- simulating the behavior of the living part in the computer system,
- calculating a maximum performance of the living part without the occurrence of an injury by the computer system,
- feeding to the system a load applied on the living part,
- calculating a current state of the living part by the computer system,
- comparing the current state of the living part with the calculated maximum performance of the living part.

By modelling, calculating and comparing the current state of a living part with its state after it has reached its maximum performance it is easy to predict if the living part is close to its failure point, thus an injury can be predicted and prevented. In other words, the comparison between current yield area and a past yield area allows hypothetic scenarios for physical improvement or deterioration.

In an example the method for injury prediction is applied on a living part which is either a soft tissue or among tendons, joints and bones. Therefore, the method can be applied for any part of a living body.

In the example that the living part is a soft tissue, it comprises a fluid part and a solid part, and the simulation of the behavior of the living part is performed by using Fluid Structure Interaction. In more detail, the simulation of the behavior of the fluid part of the soft tissue is performed by using Computational Fluid Dynamics and the simulation of the behavior of the solid part of the soft tissue is performed by using Finite Element Analysis. In this way, the simulation is more accurate since it takes into account the different components of the soft tissue, their individual behavior and synthesizes the overall fatigue behavior of the tissue.

In an example the simulation of the geometry of the living part is based on at least one Magnetic Resonance Imaging. In this way the method is totally personalized, since it uses as input the personal geometry of the living part that comes out from a Magnetic Resonance Imaging procedure.

In an example, the calculation of the maximum performance of the living part without the occurrence of an injury is based on experimental data. Experiments on animal parts, according to the respective European legislation, that resemble the behavior of other living parts, for example living human parts, offer the knowledge of when to expect the injury of the living part and prevent it.

Alternatively, the calculation of the maximum performance of the living part without the occurrence of an injury is based on Partial Differential Equations. The mathematical model offers an even more accurate prediction, in a harmless way, of the loading at which an injury may occur, offering the advantage of avoiding the injury.

In an example, the load applied on the living part comprises stresses and strains, which are either applied in an experimental setting in a laboratory or retrieved experimentally by a wearable collector of biometric data, which the athlete wears during exercise.

In an example, the feeding of the load applied on the living part is performed in real time, so the calculation of the current state of the living part is performed in real time. In addition, also the comparison of the current state of the living part with the calculated maximum performance of the living part is performed in real time. Therefore, during the loading of the living part it is possible to monitor the current state of the living part and how close it is to the failure threshold and how to avoid reaching that threshold.

When a whole team is surveyed, for example a football team, the system comprises warning mechanisms which inform the medical team graphically or by generating sounds, of possible injuries of any of the athletes. The coach then decides either to simply inform the player to pay attention to e.g. his right leg, or even to replace him by a substitute.

The method for injury prevention of living parts can be applied to any human. An example of application of the present method and system is an aged person with a track record of muscle injuries. More particularly, it is applied to high performing humans, such as professional athletes.

It is known that a possible injury may arise under the following circumstances: (a) the particular muscular system is not constructed to accept an excessive strain, (b) after a hit has occurred, (c) temporary muscular instability due to physical or mental conditions, for example anxiety, stress or illness (d) aging. So, it is even more advantageous the use of the method for predicting injuries during workout or during medical examination.

Professional athletes are valued according to their performance, age and state of health. When it is found out that e.g. a football player is injured, his price drops dramatically. As on professional sports the workouts are designed to be of high-intensity, muscle injuries are often encountered. The result is the deterioration of the athletes' health, and psychological state, as well as the time lost on inactivity, as they will need to spend time in rehabilitation. The consequent cost is related to the monetary loss of the athletes, due to their idle time and the reduction of the transfer value when they are transferred between the various athletic professional clubs. In addition, professional clubs invest large amounts of money every year in athletes, so their losses are highly related to the physical condition of the athletes during recruitment or throughout the season. Currently, injuries are well considered part of everyday practice and professional clubs take it very seriously into account, for example at the stage of training and athletes' transfer.

In the context of the present disclosure it is further described a method for material characterization of a tissue comprising the steps of:

- measuring the geometry of a specimen,
- applying forces to the specimen, the forces being controlled,
- measuring the deformation of the specimen,
- creating a diagram of elasticity and failure, through the application of a non-linear modelling protocol of hyper elasticity.

The behavior of an animal tissue can be approximated to a composite material following the rules of hyper elasticity. Consequently, the material characterization of a tissue and the creation of its elasticity diagram offer a huge amount of calculated information related to the behavior and the response of tissue to various loads.

5   EP 3 863 023 A1   6

In an example the tissue comprises elastin, fluids and muscle. The diagram of elasticity of such a tissue is correlated to its composition. In this way it is shown in a diagram the correlation between the loads and which part of the tissue is mostly overworked. The equation modelling the tissue can also calculate at which values of the load a deformation of the tissue is based on the effect of elastin.

Where the forces applied to the tissue in the lab are similar to the forces applied to the tissue during high performance activities, the method provides more realistic information regarding the state of the tissue during workout.

Obviously, the diagram of elasticity and failure is different for healthy tissue, damaged tissue and rehabilitated tissue. In this way it is easily understood the current state of a tissue and the possibility for a future failure.

In the context of the present disclosure it is described a method for creating and validating a mathematical model describing the behavior of a specific animal part. The method comprises the steps of:

- assuming a Partial Differential Equation describes or models the animal part,
- we are then varying a first parameter in the equation,
- we are further varying a second parameter in the equation,
- we are comparing the obtained results to experimental data,

and conclude that the Partial Differential Equation with the specific parameters i and j is modelling precisely this specific animal part. In this way the model of the animal part is validated, and it is more accurate, making the method for preventing injuries more accurate. The parameters i and j can also be varied simultaneously.

The Partial Differential Equation is the following:

$$W = \sum_{i+j-1}^{N} C_{ij}(I_1 - 3)^i (I_2 - 3)^j$$

wherein the first and the second parameters to be varied are i and j. In this way the equation can become specific and can result in an individual mathematical, phenomenological model for every single animal part.

In the upon equation W is strain energy density function, with dependence on $I_1, I_2, I_3$, the tree invariants of a mechanical 3D tensor (algebraical matrix).

More precisely

- $I_1$, is the invariant which refer to a matrix trace
- $I_2$, refer to sub determinant product and
- $I_3$ refer to matrix determinant.

Every tensor can be represented as principal tensor, to wit as a matrix with only diagonal elements.

That can be achieved by rotation of the random tensor. Hence W is a depended variable on $\lambda_1, \lambda_2, \lambda_2$, the tree diagonal tensor's elements after the rotation.

Living tissues are nearly incompressible so $I_3 = 1$ because the determinant refers to a body volume. According to this hypothesis w has no longer dependence on $I_3$ and $I_3$ and can be expressed by $I_1, I_2$.

C is an elasticity multiplier depending on tissue characteristics. Through changing Einstein indicators i and j we can derive several mechanical models describing mostly the distribution of elastin fibers. These models constitute a strong indication of how loads can be applied and where lie the limits of the tissue.

$$I_1 = \lambda_1^2 + \lambda_2^2 + \lambda_3^2$$

$$I_2 = \lambda_1^2 \lambda_2^2 + \lambda_3^2 \lambda_2^2 + \lambda_1^2 \lambda_3^2$$

$$I_3 = \lambda_1^2 \lambda_2^2 \lambda_3^2 = 1$$

Consequently, every single animal part is simulated, stored in a computer memory and its behavior, therefore its failures or injuries can be predicted with high accuracy.

As one can expect, the equation modelling the animal part is a non-linear equation, as the structure of each animal part is non-linear, and every part needs to respond to a plurality of non-linear loads.

The equation modelling the tissue calculates at which values of the load a deformation of the muscle reaches the upper limit and the tissue is close to be injured. These mathematical models are named after the parts kinematics that they describe, for example Hamstring Load, ACL load etc.

The living parts of the present invention are mainly but not exclusively soft tissues, comprising a fluid part and a solid part. Nevertheless, the living part can also be among tendons, joints and bones.

The simulation of the geometry of the living part is based on at least one Magnetic Resonance Imaging scan of the animal, such as the athlete.

The simulation of the behavior of the living part is performed by using Fluid Structure Interaction, the simulation of the behavior of the fluid part of the soft tissue is performed by using Computational Fluid Dynamics and the simulation of the behavior of the solid part of the soft tissue is performed by using Finite Element Analysis.

The method hereby is performed by a system comprising means for experimental material characterization of an animal part by using non-linear modeling of hyper elasticity, a mathematical model using Partial Differential Equations validated by the experimental material characterization, a computational model of the animal part validated by the experimental and the mathematical modelling, wherein the computational model compares

4

a current state of the animal part with a calculated future state of the animal part after having performed at its maximum potential.

Furthermore, the method for material characterization of a tissue can also comprise the steps of:

- measuring the geometry of a specimen and storing it in a computer,
- applying forces to the specimen, the forces being controlled,
- measuring the deformation of the specimen,
- creating a diagram of elasticity and failure, wherein the method applies a non-linear modelling protocol of hyper-elasticity.

Obviously, the method can be applied to high performing athletes, such as football, basketball, tennis players, but can also be applied to animals of high commercial value, such as horses.

**Brief description of the drawings**

The description that follows, will be better understood when read in conjunction with the figures shown. For purposes of illustration, the figures show specific embodiments of the present disclosure. However, it should be understood that the present disclosure is not limited to the particular embodiment and the features appearing. The accompanying drawings, which are included and form part of the description, illustrate the embodiment of apparatus according to the present description and, together with it, serve to illustrate advantages and principles in accordance with the present description.

Aiming at a better understanding of the invention, its application in the field of soccer will be used herein and after as an example. However, a person skilled in the art can understand that the present method can have a more extensive application, for example it can be widely used in any sport, in medical centers where people are exercising in order to be examined, for example during a stress echocardiography test, or it can be the basis for the development of more sophisticated artificial body parts.

Fig. 1 represents the experimental apparatus used in the development of the method
Fig. 2 shows two diagrams comparing the experimental results with the results from the mathematical model
Fig. 3 shows a graphical representation of the area where the animal part has the highest potential for injury
Fig. 4 shows a Magnetic Resonance Imaging scan of the living part
Fig. 5 shows a computational ANSYS 3D model of a living part
Fig. 6 shows examples of sports wearables
Fig. 7 shows the computed stress and strain on a mechanical model of a living part
Fig. 8 shows a 2D stress-strain diagram of the current state of a group of living parts
Fig. 9 represents a comparison of the 2D stress-strain diagram of the current state with the graphical representation of the area where the animal part has the highest potential for injury
Fig. 10 shows a different image of the areas of the living part with the higher possibility for injury.
Fig. 11A shows an overview of the team in terms of the position of each soccer player and his/her fatigue index
Fig. 11B shows the current fatigue state of a single soccer player
Fig. 11C shows a screen of the application focusing on one tissue of a single soccer player as well as suggestions

The following description of the invention is made with reference to the accompanying drawings, where the same reference numbers denote identical or similar elements.

**Detailed Description**

The phrase and terminology used below are for the purpose of description and should not be construed as limiting. For example, using a single grade, such as "one" does not intend to determine the number of operations. In addition, it is to be understood that any of the features of the present disclosure may be used autonomously or in combination with other features. Other functionally equivalent systems, methods, features, uses, and advantages of the present disclosure will be apparent to one skilled in the art after examining the figures and description. All additional systems, methods, features, uses and advantages are intended to be within the scope of the present disclosure and be protected from the scope of the accompanying claims.

In the present document it is disclosed the development and the use of a method for injury prevention of living parts. The initial step in the process of development is the characterization of the living part through an experimental method. It is known that the living parts have the behavior of a composite material with unknown mechanical properties. The outcome of the experimental method is to characterize the complex bio-material. This procedure leads to information about the mechanical behavior of a tissue in tension, extension, rotation in all three directions.

Based on bibliography, the response to loading of soft tissues belonging to pigs is similar to the response of some of the human soft tissues, for example the biceps. Using BioEngineering protocols we implement experiments to soft tissues so as to accomplish a characterization of the material. These protocols are known in the art and described in "An Experimental Model on the Biomechanical Behavior of the Flexor Tendons in New Zealand Rabbits" (The Journal of Hand Surgery (Asian-Pacific Volume) 2017;22(3):1-9).

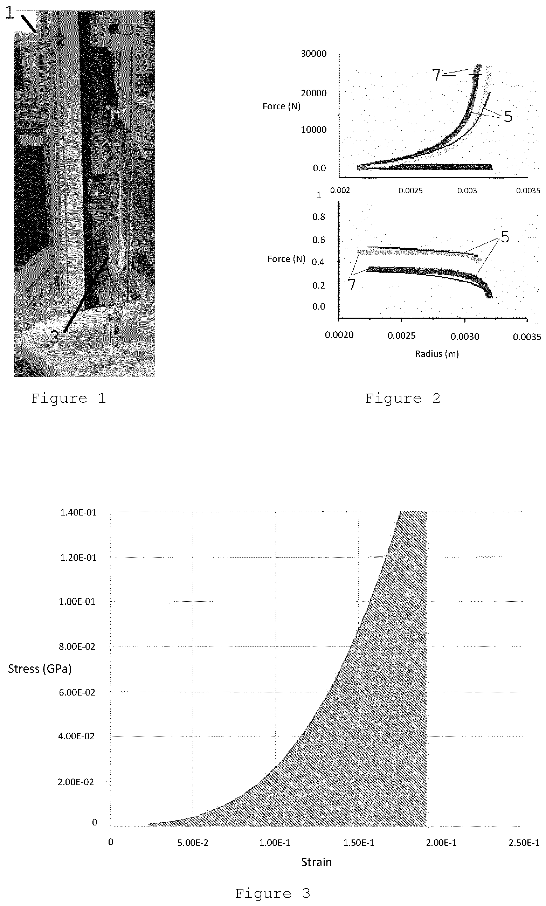

In fig.1 it is shown the experimental apparatus 1, where loads are applied on the uninjured specimen 3. In order to simulate the loading of the tissue when an animal is moving, the loading follows a non-linear pattern in all axes. During the experiments it is measured the deformation of the specimen 3 in all axes while it is under load, and the elasticity diagram of the soft tissue is prepared. The stress strain curve is performed.

Additionally, from bibliography it is known that a living tissue comprises fluids, elastin fibers, collagen and muscle. Also, during the analysis of the experimental data, some assumptions were made, among which that the soft tissue material is responding to loads in accordance to non-linear models of hyper elasticity. This assumption, in correlation to the experimental data and using as basis the way of extracting the stress-strain relationship of hyper elastic soft

materials as it is described in bibliography in "Extracting the isotropic uniaxial stress-strain relationship of hyper elastic soft materials based on new nonlinear indentation strain and stress measure", (Zhang, Q., Li, X., Yang, Q., AIP Advances 8, 115013 (2018)), leads to the conclusion that the tissue as a material corresponds indeed to a complex material of hyper elasticity. Also, based on the produced elasticity diagram, and by knowing the mechanical properties of a tissue, it is easy to understand, according to the loading, if the deformation of the tissue is in the zone where the elastin is mainly responding or it is in the zone where the muscle deforms and what the loading should be in order for the muscle to reach its failure point and to be injured.

Although the experimental part was based on the use of specimen of uninjured soft tissue, a person skilled in the art can understand that similar experiments can be conducted for other animal parts, for example joints, bones and tendons. In addition, similar experiments may focus on injured or rehabilitated animal parts, since, according to bibliography, the mechanical properties and the response to loading of such animal parts is different from the ones of the healthy animal parts. These experiments would lead to the characterization of the material of the mentioned animal parts and their mechanical properties, leading to different elasticity and failure diagrams.

The next step in the development of the method is building a mathematical model of an animal part. As the problem has no analytical solution, numerical analysis is used, designing a mathematical model to explain the load distribution. It is assumed that the bio- material follows mathematical formulation as everything in nature. In the art it is known that the equation

$$W=W(I1,I2,I3)=W(I1,I2,j)$$

or

$$W=W(\lambda1,\lambda2,\lambda3)$$

where W is the strain energy density, dependent on the three invariant mechanical tensors I1, I2, I3. Assuming that j=1 due to the material being incompressible, the strain energy function depends only on I1 and I2 and consequently on $\lambda1$, $\lambda2$, $\lambda3$ tensor eigenvalues.

C is a multiplier dependent on elastin's orientation. Then,

$$I_1 = \lambda_1^2 + \lambda_2^2 + \lambda_3^2$$

$$I_2 = \lambda_1^2\lambda_2^2 + \lambda_3^2\lambda_2^2 + \lambda_1^2\lambda_3^2$$

$$I_3 = \lambda_1^2\lambda_2^2\lambda_3^2 = 1$$

becomes

$$W = \sum_{i+j-1}^{N} \quad C_{ij}(I_1 - 3)^i (I_2 - 3)^j$$

can represent the behavior of a tissue when the parameters i and j, the Einstein indices, have a specific value. It is assumed that when the parameters i and j are assigned different values, the above equation can represent mathematically the behavior of an animal part. By using a computer program thousands of alterations and combinations of different values for the parameters i and j are tried. The result of every alteration is compared to the experimental results . In that way it is concluded that the above equation can represent the behavior of animal parts, for example soft tissue, when the i and j are assigned a specific value. In the example of soccer, when i=... and j=... the equation represents the behavior of the human biceps when they are loaded, for example when an athlete is walking or sprinting.

In Fig. 2 it is shown the diagram resulting from the application of various loads to the equation above. The lines 5 show the results of the experimental process while the other lines 7 derived from the mathematical model for the example of the biceps. The results from the two models coincide, thus the validity of the mathematical model is evident. Although fig. 2 represents only the above-mentioned example, it is understandable that similar diagrams can be produced for other animal parts.

Based on the mathematical model and the experimental model it is possible to calculate a stress-strain area where the possibility of injuring the animal parts is the highest. The schematic representation of that area is shown in fig. 3.

Having in place two models, the next stage in the development of the method for injury prevention of living parts is the creation of a computational model. The basis for this model is the comparison of the current state of the living part with the future state of the living part after it has reached its maximum performance, since it is known that a living part reaches its failure point depending on its current state, its maximum potential and the loads exerted on it.

The steps to be followed are: the simulation of the geometry of the living part, the input of the material characteristics and its mechanical properties, as these were found experimentally, the virtual application of loads exerted on the living part based on real data, the calculation of the current state of the living part, the calculation of the future state of the living part after it has reached its maximum performance and the comparison of the two states.

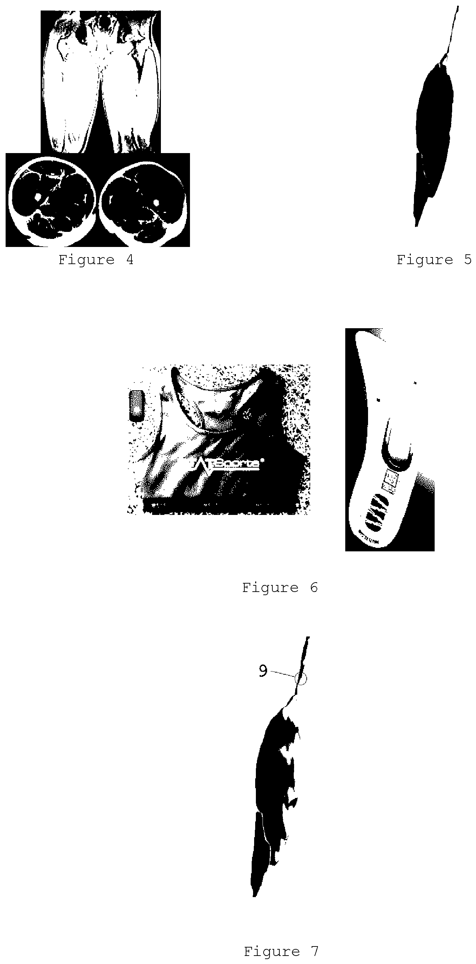

In order to simulate the geometry of the living part a commercial software (ANSYS) is employed, customized with FORTRAN90 and PYTHON functions. A shown in fig. 4, a Magnetic Resonance Imaging scan of the living part offers detailed input regarding the geometry of the living part. When the image from the Magnetic Resonance Imaging scan of the living part is input in ANSYS, the system builds layer by layer a model of the living part. The final model is shown in fig. 5.

The material characteristics are also used as input in the system, as well as the fact that the living part may be a human tissue and it comprises a fluid part and a solid muscle part. Due to the complexity of the material and the need for the results to be realistic, the fluxes of the internal areas of the living part were examined with the use of Computational Fluid Dynamics methods, while the external area was examined with the use of Finite Element Analysis. The combination of the two methods is known as Fluid Structure Interaction (FSI).

Additional required input was data related to the realistic loading in all axes of the living parts. In the example were this method is applied to soccer or other sports, this kind of data can be retrieved by pod-carrying vests, like the one commercially available by Stat-Sports®, or any other kind of wearables with sensors, all shown in fig. 6. These wearables provide information related to the acceleration of an athlete, the distance travelled and the load on the left and the right leg. Also, this data may be retrieved at any time, even in real time, helping in the immediate prevention of injuries. This example is related to soccer, so this information is enough for the prevention of injuries, since the soccer players are mostly injured on the legs. However, if this method is to be applied in any other sport, for example in basketball, or during a medical examination, the loading input required may be different and it can be retrieved by other type of wearables.

Since both the modelling of the living part and the data of the loads exerted on the living parts are different for different individuals, the representation of the living part and its state is individual for every living part and for every animal or human having this living part.

Having inserted all this input in the system, the loads are applied on the living parts and it creates a mechanical analysis of the living parts' stress and strain, which is shown in fig. 7. Although it is not so obvious in gray scale, the most stressed area is 9, and an athlete bears the higher possibility to be injured at this area. This shows the current state of the living part.

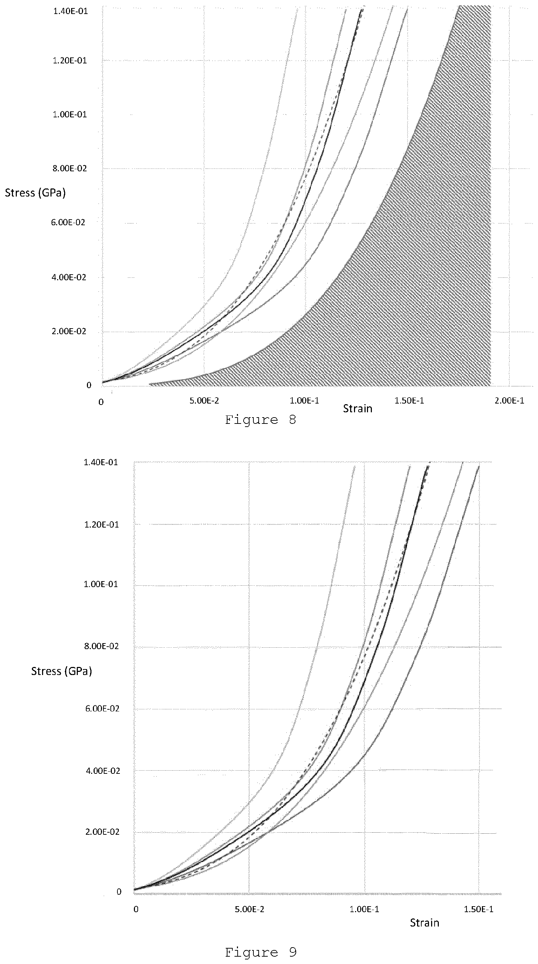

The current state of the living part can be also translated to a 2D stress-strain diagram, shown in fig. 8. In this diagram there are various lines, each showing the current state of the same kind of a living part, for example of the left bicep, belonging to different soccer players. This diagram can be compared to the schematic representation of the area where injury is likely to occur and is shown in fig. 3. The result is the comparative diagram of fig. 9.

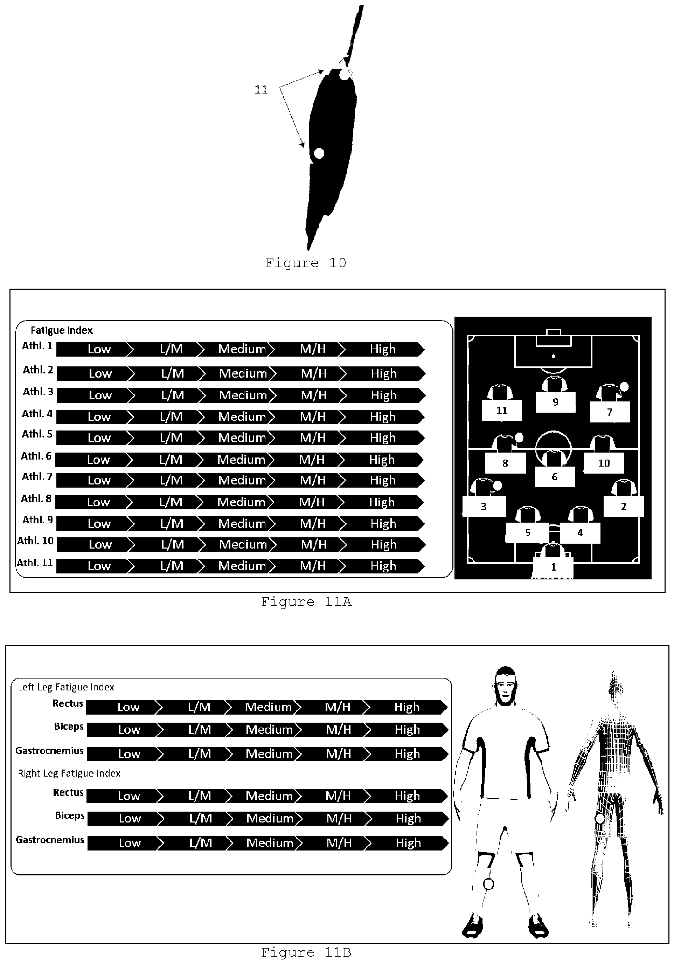

In addition, another image may be produced by the system, shown in fig. 10, where the areas 11 bear the higher possibility of injury.

By using the diagram of fig.9 and the image of fig. 10, a person responsible for medical treatment and/or management, for example a doctor, can understand the possibility of injury for a person and prevent it by altering his/her workload routine.

Apart from the data described above, the system may be able to provide additional information regarding the current and the future state of a living part, for example a table with personal data of the person under examination, in the soccer example the player, the person's muscular system details like the exerted stress or strain, an injury index potential, suggested actions, personalized impact of the loads on the living parts per time etc.

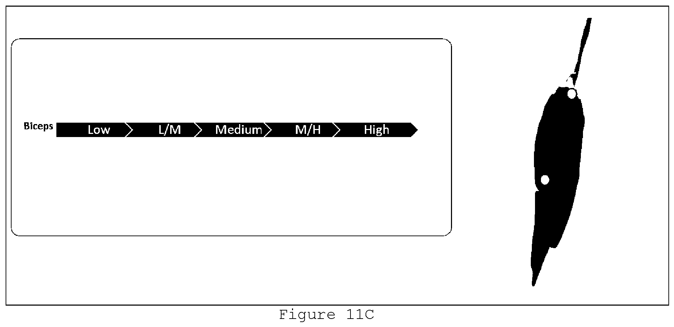

Although the application of the method requires a significant computational power, it is possible for the results to be sent in an application for a mobile device, for example a mobile phone or a tablet. This would be very advantageous in the example of soccer, when the data of the players that are collected by the wearables are sent in real time to the system. Before a soccer game, the living parts, like the biceps, of the athletes may be modelled and during a soccer game the loading data may be sent in real time to the system. The system can then compute the current state of the living parts per athlete and send back to the coach the situation of every athlete, a prediction of the player's possibility to be injured due to the failure of his/her biceps, as well as suggestions for actions to be taken. Examples of the visual representation as seen from the coach's tablet are shown in figs. 11A to 11C.

## Claims

1. A method for injury prediction of living parts comprising the steps of:

- simulating the geometry of a living part in a computer system,
- simulating the behavior of the living part in the computer system,

- calculating a maximum performance of the living part without the occurrence of an injury by the computer system,
- feeding to the system a load applied on the living part,
- calculating a current state of the living part by the computer system,
- comparing the current state of the living part with the calculated maximum performance of the living part.

2. A method for injury prediction of living parts according to claim 1, wherein the living part is a soft tissue comprising a fluid and a solid part.

3. A method for injury prediction of living parts according to claim 1, wherein the living part is among tendons, joints and bones.

4. A method for injury prediction of living parts according to claim 1, wherein the simulation of the geometry of the living part is based on at least one Magnetic Resonance Imaging scan.

5. A method for injury prediction of living parts according to claim 1, wherein the simulation of the behavior of the living part is performed by using Fluid Structure Interaction.

6. A method for injury prediction of living parts according to claims 3 to 5, wherein the simulation of the behavior of the fluid part of the soft tissue is performed by using Computational Fluid Dynamics.

7. A method for injury prediction of living parts according to claims 2 to 5, wherein the simulation of the behavior of the solid part of the soft tissue is performed by using Finite Element Analysis.

8. A method for injury prediction of living parts according to claim 1, wherein the calculation of the maximum performance of the living part without the occurrence of an injury is based on a Partial Differential Equation.

9. A method for injury prediction of living parts according to claim 1, wherein the calculation of the maximum performance of the living part without the occurrence of an injury is based on experimental data.

10. A method for injury prediction of living parts according to claim 9, wherein the load applied on the living part comprises stress and/or strain.

11. A method for injury prediction of living parts according to claim 10, wherein the load applied on the living part is measured experimentally in real time by a wearable collector of biometric data.

12. A method for injury prediction of living parts according to claims 1 or 11, wherein the calculation of the current state of the living part is performed in real time.

13. A method for injury prediction of living parts according to any of claims 1, 11 and 12, wherein the comparison of the current state of the living part with the calculated maximum performance of the living part is performed in real time.

14. Use of a method for injury prediction of living parts of claim 1 to a high performing human.

15. A system for injury prediction of living parts comprising:

- Means for experimental material characterization of an animal part by using non-linear modeling of hyper elasticity,
- a mathematical model simulating a living part using Partial Differential Equations validated by the experimental material characterization,
- a computational model of the animal part validated by the experimental and the mathematical modelling,

wherein the computational model compares a current state of the animal part with a calculated future state of the animal part after having performed at its maximum potential.

Figure 1

Figure 2

Figure 3

Figure 4

Figure 5

Figure 6

Figure 7

Figure 8

Figure 9

Figure 10

Figure 11A

Figure 11B

Figure 11C

**EUROPEAN SEARCH REPORT**

Application Number

EP 20 02 0060

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | REHORN M R ET AL: "The effects of aponeurosis geometry on strain injury susceptibility explored with a 3D muscle model", JOURNAL OF BIOMECHANICS, PERGAMON PRESS, NEW YORK, NY, US, vol. 43, no. 13, 17 September 2010 (2010-09-17), pages 2574-2581, XP027267065, ISSN: 0021-9290 [retrieved on 2010-06-11] | 1,14 | INV. G16H50/30 |
| Y | * Introduction, Discussion, Abstract * ----- | 1,2,4-14 | |
| Y | JOSHUA INOUYE ET AL: "Fiber tractography for finite-element modeling of transversely isotropic biological tissues of arbitrary shape using computational fluid dynamics", PROCEEDINGS OF THE CONFERENCE ON SUMMER COMPUTER SIMULATION, 26 July 2015 (2015-07-26), pages 1-6, XP055714244, ISBN: 978-1-5108-1059-4 * Introduction, Discussion, Abstract * ----- | 1,2,4-14 | |
| Y | Joshua Inouye ET AL: "MUSCLE ARCHITECTURE ANALYSIS USING COMPUTATIONAL FLUID DYNAMICS", , 1 January 2015 (2015-01-01), XP055714412, Retrieved from the Internet: URL:https://asbweb.org/conferences/2015/abstracts/PD1E_6--Muscle%20Architecture%20Analysis%20Using%20Computational%20Fluid%20Dynamics--(Inouye).pdf [retrieved on 2020-07-14] * Introduction, Discussion, Abstract * ----- -/-- | 1,2,4-14 | |

TECHNICAL FIELDS SEARCHED (IPC)

G16H

~~The present search report has been drawn up for all claims~~

| Place of search | Date of completion of the search | Examiner |
|---|---|---|
| Munich | 16 July 2020 | Vogt, Titus |

page 1 of 4

| | Europäisches Patentamt European Patent Office Office européen des brevets | **EUROPEAN SEARCH REPORT** | Application Number EP 20 02 0060 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AMANDA M. WESTMAN ET AL: "A coupled framework of in situ and in silico analysis reveals the role of lateral force transmission in force production in volumetric muscle loss injuries", JOURNAL OF BIOMECHANICS, vol. 85, 1 March 2019 (2019-03-01), pages 118-125, XP055714246, US ISSN: 0021-9290, DOI: 10.1016/j.jbiomech.2019.01.025 | 1,2,14 | |
| Y | * Introduction, Discussion, Abstract * ----- | 1,2,4-14 | |
| Y | GUOAN LI ET AL: "The Effect of Anterior Cruciate Ligament Injury on Knee Joint Function under a Simulated Muscle Load: A Three-Dimensional Computational Simulation", ANNALS OF BIOMEDICAL ENGINEERING, vol. 30, no. 5, 1 May 2002 (2002-05-01), pages 713-720, XP055714013, New York ISSN: 0090-6964, DOI: 10.1114/1.1484219 * Introduction, Discussion, Abstract * ----- | 1,2,4-14 | |
| | | | TECHNICAL FIELDS SEARCHED (IPC) |
| X | VICKIE B. SHIM ET AL: "Subject-specific finite element analysis to characterize the influence of geometry and material properties in Achilles tendon rupture", JOURNAL OF BIOMECHANICS, vol. 47, no. 15, 1 November 2014 (2014-11-01), pages 3598-3604, XP055714015, US ISSN: 0021-9290, DOI: 10.1016/j.jbiomech.2014.10.001 | 1,14 | |
| Y | * Introduction, Discussion, Abstract * ----- -/-- | 1,4-14 | |

~~The present search report has been drawn up for all claims~~

| Place of search | Date of completion of the search | Examiner |
|---|---|---|
| Munich | 16 July 2020 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches
Patentamt

European
Patent Office

Office européen
des brevets

**EUROPEAN SEARCH REPORT**

Application Number

EP 20 02 0060

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALFIO QUARTERONI ET AL: "Computing and Visualization in Science Computational vascular fluid dynamics: problems, models and methods", COMPUT VISUAL SCI, vol. 2, 1 January 2000 (2000-01-01), pages 163-197, XP055714016, | 1,2,14 | |
| Y | * Introduction, Discussion, Abstract * | 1,2,4-14 | |
| X | MIR-HOSSEIN MOOSAVI ET AL: "Numerical simulation of blood flow in the left ventricle and aortic sinus using magnetic resonance imaging and computational fluid dynamics", COMPUTER METHODS IN BIOMECHANICS AND BIOMEDICAL??ENGINEERING, vol. 17, no. 7, 13 September 2012 (2012-09-13), pages 740-749, XP055714170, GB ISSN: 1025-5842, DOI: 10.1080/10255842.2012.715638 | 1,2,14 | |
| Y | * Introduction, Discussion, Abstract * | 1,2,4-14 | |
| Y | TIEN TUAN DAO ET AL: "A Systematic Review of Continuum Modeling of Skeletal Muscles: Current Trends, Limitations, and Recommendations", APPLIED BIONICS AND BIOMECHANICS, vol. 2018, 6 December 2018 (2018-12-06), pages 1-17, XP055714544, Cambridge ISSN: 1176-2322, DOI: 10.1155/2018/7631818 * Whole document, Tables * | 1,2,4-14 | TECHNICAL FIELDS SEARCHED (IPC) |

-/--

~~The present search report has been drawn up for all claims~~

| Place of search | Date of completion of the search | Examiner |
|---|---|---|
| Munich | 16 July 2020 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 4

Europäisches Patentamt

European Patent Office

Office européen des brevets

# EUROPEAN SEARCH REPORT

Application Number

EP 20 02 0060

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YUJIANG XIANG ET AL: "Computational Methods for Skeletal Muscle Strain Injury: A Review", CRITICAL REVIEWS IN BIOMEDICAL ENGINEERING., vol. 47, no. 4, 1 January 2019 (2019-01-01), pages 277-294, XP055714562, US ISSN: 0278-940X, DOI: 10.1615/CritRevBiomedEng.2019029194 * Whole document, Tables * | 1,2,4-14 | |
| X | NICCOLO M. FIORENTINO ET AL: "Computational Models Predict Larger Muscle Tissue Strains at Faster Sprinting Speeds :", MEDICINE AND SCIENCE IN SPORTS AND EXERCISE, vol. 46, no. 4, 1 April 2014 (2014-04-01), pages 776-786, XP055714574, US ISSN: 0195-9131, DOI: 10.1249/MSS.0000000000000172 | 1,14 | |
| Y | * Introduction, Discussion, Abstract * | 1,2,4-14 | |
| X | US 2015/080766 A1 (JI SONGBAI [US] ET AL) 19 March 2015 (2015-03-19) | 1,14 | |
| Y | * paragraph [0075]; claims; figures * | 1,2,4-14 | |
| X | WO 2017/025979 A1 (ANSHUMAN KUMAR SINGH [IN]) 16 February 2017 (2017-02-16) | 1,2,14 | |
| Y | * paragraphs [0020], [0023], [0026], [0065], [0066]; claims; figures * | 1,2,4-14 | |

TECHNICAL FIELDS SEARCHED (IPC)

~~The present search report has been drawn up for all claims~~

| Place of search | Date of completion of the search | Examiner |
|---|---|---|
| Munich | 16 July 2020 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

Europäisches
Patentamt

European
Patent Office

Office européen
des brevets

**Application Number**

EP 20 02 0060

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

2(completely); 1, 4-14(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 20 02 0060

---

The Search Division considers that the present European patent application does not comply with the
requirements of unity of invention and relates to several inventions or groups of inventions, namely:

1. claims: 2(completely); 1, 4-14(partially)

    A method of preventing injury of a soft tissue comprising
    fluid and a solid.
    ---

2. claims: 1, 3-14(all partially)

    A method of preventing injury of a tendon
    ---

3. claims: 1, 3-14(all partially)

    A method of preventing injury of joints
    ---

4. claims: 1, 3-14(all partially)

    A method of preventing injury of bones
    ---

5. claim: 15

    A system for injury prediction.
    ---

## ANNEX TO THE EUROPEAN SEARCH REPORT
## ON EUROPEAN PATENT APPLICATION NO.

EP 20 02 0060

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2020

| Patent document cited in search report | Publication date | Patent family member(s) | Publication date |
|---|---|---|---|
| US 2015080766 A1 | 19-03-2015 | NONE | |
| WO 2017025979 A1 | 16-02-2017 | US 2018235517 A1<br>WO 2017025979 A1 | 23-08-2018<br>16-02-2017 |

EPO FORM P0459

For more details about this annex : see Official Journal of the European Patent Office, No. 12/82

## REFERENCES CITED IN THE DESCRIPTION

**Patent documents cited in the description**

- US 2019313934 A
- WO 2019097166 A
- CN 203736191
- CN 202235414
- EP 0761166 A
- CN 103926169

**Non-patent literature cited in the description**

- Sports Medicine Statistics, An Issue of Clinics in Sports Medicine. **HART,J.M ; THOMPSON, S.R.** Sports Medicine Statistics, An Issue of Clinics in Sports Medicine. Elsevier, 2018
- An Experimental Model on the Biomechanical Behavior of the Flexor Tendons in New Zealand Rabbits. *The Journal of Hand Surgery (Asian-Pacific Volume),* 2017, vol. 22 (3), 1-9
- **ZHANG, Q. ; LI, X. ; YANG, Q.** Extracting the isotropic uniaxial stress-strain relationship of hyper elastic soft materials based on new nonlinear indentation strain and stress measure. *AIP Advances,* 2018, vol. 8, 115013